## Europäisches Patentamt

(19) **European Patent Office**

**Office européen des brevets**

(11) Numéro de publication: **0 1 1 7 859**
**B1**

---

(12) **FASCICULE DE BREVET EUROPEEN**

(45) Date de publication du fascicule du brevet:
**24.06.87**

(21) Numéro de dépôt: **84870004.3**

(22) Date de dépôt: **06.01.84**

(51) Int. Cl.⁴: **A 61 B 17/60**

---

(54) Procédé de contrôle de la stabilité d'un montage orthopédique constitué d'une barre de fixation externe utilisée pour la réduction des fractures.

---

(30) Priorité: **28.01.83 BE 209986**

(43) Date de publication de la demande:
**05.09.84 Bulletin 84/36**

(45) Mention de la délivrance du brevet:
**24.06.87 Bulletin 87/26**

(84) Etats contractants désignés:
**AT BE CH DE FR GB IT LI LU NL SE**

(56) Documents cités:
**FR - A - 1 569 090**
**FR - A - 2 429 586**
**US - A - 3 866 607**

**ELECTROTECHNOLOGY, vol. 4, no. 4, octobre 1976, pages 3-6, The Institution of Electrical and Electronics Technician Engineers, Londres, GB R.F. RUSS: "Strain-gauges and strain-gauged devices"**

(73) Titulaire: **Région Wallonne représentée par l'Exécutif Régional Wallon, 11, Boulevard de l'Empereur, B-1000 Bruxelles (BE)**

(72) Inventeur: **Boland, Michel Armand Ghislain, Chemin de la Belle Epine, 20, B-6428 Ham-sur-Heure (BE)**

(74) Mandataire: **Blanchart, André, MINISTERE DE LA REGION WALLONE Direction d'Administration de l'Energie et des Technologies nouvelles Service des Technologies nouvelles Avenue Prince de Liège, 7, B-5100 Namur (BE)**

---

ACTORUM AG

## Description

L'invention se rapporte à un procédé de contrôle de la stabilité d'un montage orthopédique utilisé pour la réduction des fractures constitué d'une barre de fixation externe pourvue de jauges de contrainte pour la mesure des efforts dans le système externe. Les systèmes de fixation externe utilisés en chirurgie osseuse sont destinés au maintien de la réduction interfragmentaire pendant la phase de consolidation de la fracture ou de l'ostéotomie. Ces systèmes offrent une liaison mécanique avec les leviers osseux et le milieur extérieur. Ce montage orthopédique comprend une ou plusieurs barres de fixation reliées à au moins deux broches par l'intervention d'étaux, les broches étant fixées par des moyens connus aux fragments osseux en cas de fracture.

Actuellement, les moyens principalement utilisés en médecine pour évaluer la consolidation d'une fracture sont la radiographie et l'évaluation manuelle de la rigidité. D'autres méthodes, telles que l'examen histologique, l'étude par marquage isotopique, les méthodes vibratoires et l'utilisation des potentiels électriques de l'os n'ont pas dépassé le stade de l'expérimentation. Toutefois, aucune de ces méthodes ne permet actuellement de mesurer directement l'évolution de la résistance mécanique de l'os, ni de contrôler la stabilité mécanique des montages d'ostéosynthèse lors de sollicitations ou de mouvements de revalidation. Il est également connu d'évaluer la reconsolidation au moyen de barres de fixation externes équipées d'une seule jauge de contrainte ohmique. Toutefois l'utilisation d'une seule jauge rend la méthode dépendante de l'habilité du chirurgien à orienter cette jauge selon un plan de déformation maximale car seule une composante de la flexion est déterminée. Cette technique ne permet que des mesures relatives de l'évolution de la reconsolidation de la fracture.

D'autre part il est apparu que la rééducation des patients est ralentie par la peur d'entraîner une nouvelle fracture par une surcharge dépassant la résistance du cal et de la fixation, et provoquant simultanément des glissements irréversibles dans les étaux et des déplacements des fragments osseux dans le foyer de fracture.

La présente invention a pour objet la détermination objective de la torsion et de la flexion de la barre de fixation externe au niveau des deux étaux les plus proches, situés sur la barre, de part et d'autre de la partie jaugée.

Il est ainsi possible d'évaluer le risque de glissement dans ces étaux en comparant les efforts déterminés aux efforts pouvant être supportés par ces étaux.

La présente invention se rapporte à un procédé de contrôle de la stabilité d'un montage orthopédique effectué à l'aide d'une fixation externe et comprenant au moins une barre de fixation reliée à au moins deux broches au moyen d'étaux, caractérisé par l'utilisation d'une barre de fixation pourvue de 5 ensembles de jauges de contrainte d'au moins une jauge chacun, un ensemble mesurant la torsion de la barre et 2 paires d'ensembles mesurant deux composantes perpendiculaires de la flexion de la barre en deux points de celle-ci, par la détermination à l'aide de ces jauges de contrainte de la torsion et de la flexion au niveau des étaux qui relient la barre de fixation aux broches et par la comparaison des niveaux de torsion et de flexion déterminés à ceux pouvant provoquer des glissements dans les étaux.

Le montage orthopédique effectué à l'aide de la fixation externe peut comprendre une ou plusieurs barres de fixation. Ces barres peuvent être d'un type quelconque. Elles sont reliées par au moins deux broches aux fragments osseux. Le montage des barres peut comprendre des interconnexions.

Par étau, on comprend tout dispositif mécanique reliant les différents éléments de la fixation entre eux.

Par jauge de contrainte, on comprend tout dispositif d'un type connu utilisé pour la mesure des contraintes mécaniques. Elle comprend par exemple, tout dispositif dont la résistance électrique varie en fonction de son allongement, et permet ainsi de déterminer l'allongement d'un élément dont elle serait solidaire.

Les cinq ensembles de jauges de contrainte d'au moins une jauge chacun sont nécessaires et suffisants pour mesurer les sollications en glissement des étaux. Il n'est pas exclu d'utiliser des ensembles supplémentaires remplissant d'autres fonctions, par exemple mesurer des contraintes en traction-compression.

La disposition revendiquée détermine au moyen du premier ensemble la valeur de la torsion, qui est constante jusqu'aux deux étaux les plus proches situés de part et d'autre du point de la mesure, et au moyen de deux autres paires d'ensembles, la flexion le long de la barre et donc au niveau de ces étaux, grâce à la connaissance en deux points de son évolution linéaire.

Suivant une réalisation préférentielle du procédé revendiqué, chaque ensemble de jauges de contrainte comprend de 1 à 4 jauges. Les mesures s'effectuant habituellement par des points de Wheatstone, qui comprennent chacun 4 branches, il n'est pas efficace d'utiliser plus de 4 jauges par ensemble. Toutefois, il n'est pas exclu d'utiliser plus de 4 jauges par ensemble. Dans le cas d'utilisation d'un ensemble comportant une seule jauge de contrainte pour la mesure de la torsion, des corrections doivent être apportées pour tenir compte que la jauge de torsion est également soumise à d'importants efforts de flexion. La mesure des déformations au niveau des jauges est obtenue en reliant les jauges de contrainte à tout appareil de mesure de type connu, tel que les ponts de mesure, et équipé d'un commutateur permettant la mesure rapide de plusieurs groupes de jauges. Par exemple, on peut utiliser le pont de mesure DMD 20 et le commutateur UMK 10 de HOTTINGER BALDWIN MESSTECHNIK pour des mesures statiques et l'installation automatique de commutation et de mesure type UPH 3200 de HOTTINGER BALDWIN MESS-TECHNIK pour des mesures dynamiques.

La détermination de la torsion et des flexions au niveau des deux étaux les plus proches, situés sur la barre de part et d'autre de la partie jaugée, se fait par calcul au départ des mesures décrites ci-dessus. La torsion étant constante le long d'une barre droite, entre deux points d'ancrage, l'effort calculé au niveau des jauges peut être directement comparé à la valeur limite provoquant le glissement de la barre dans les deux étaux les plus proches situés sur la barre de part et d'autre du point de mesure.

La flexion et ses composantes évoluant de façon linéaire le long d'une barre droite, entre deux points d'ancrage, il est nécessaire de connaître les distances entre les points de mesure et les étaux les plus proches situés de part et d'autre des points de mesure. On peut ainsi extrapoler la valeur des composantes de la flexion au niveau de ces étaux, y recomposer la flexion et comparer les valeurs obtenues aux valeurs limites provoquant le glissement des étaux.

Les efforts limites des étaux sont des valeurs qui sont déterminées statistiquement, pour un type donné d'étau, par des essais sur machine de traction par exemple.

Suivant la présente invention, la détermination de la torsion et de la flexion au niveau des étaux, et la comparaison de leur valeur aux valeurs pouvant provoquer des glissements dans les étaux sont effectuées en continu, à l'aide d'un appareil de mesure et d'interprétation des résultats par programme logiciel.

L'invention sera mieux comprise à l'aide de l'exemple non limitatif suivant. La barre, équipée de 5 ensembles de deux jauges chacun est connectée à un premier petit boîtier; celui-ci contient les résistances de haute précision nécessaires pour compléter les cinq ensembles de jauges de contrainte afin de constituer des ponts de Wheaststone. Les signaux électriques recueillis aux bornes de ces ponts sont amplifiés par des amplificateurs à haute performance et faible dérive, puis transmis à un deuxième boîtier. Ici, les cinq signaux sont convertis l'un après l'autre en un code digital. L'électronique de ce boîtier comprend un dispositif de commutation qui scrute consécutivement chaque signal électrique analogique et le transmet à un convertisseur analogique digital de grande dynamique. Un microprocesseur assure la logique d'adressage et de sélection de canal, ainsi que le stockage transitoire, le codage et la sérialisation de l'information. A la sortie de ce boîtier, l'information digitale est transmise à un centre de traitement, soit par câble, soit par ondes hertziennes, soit par tout autre moyen de transmission. Le dispositif de traitement est un micro-ordinateur qui possède le logiciel nécessaire au décodage de l'information et au calcul de la valeur de la torsion et de la flexion. Ces valeurs sont comparées aux valeurs limites provoquant des glissements dans les étaux. Cette comparaison est effectuée au fur et à mesure que l'information est acquise. Le procédé de contrôle de la stabilité d'un montage orthopédique suivant la présente invention peut comprendre des dispositifs visuels ou sonores avertissant l'opérateur en cas de dépassement des limites des contraintes imposées.

Il est possible, dans l'exemple de réalisation décrit ci-dessus, de programmer le micro-ordinateur pour commander un affichage sur l'écran cathodique, ou pour enclencher un signal sonore via un relais quand une limite jugée raisonnable par l'utilisateur est dépassée. Suivant le procédé revendiqué, la barre de fixation pourvue de 5 ensembles de jauges de contrainte permet également de suivre, dans le temps, l'évolution de la consolidation de la fracture. Lors de sollicitations de référence de l'ensemble du montage osmatériel d'ostéosynthèse, on mesure la part des forces reprises par la barre elle-même.

En répétant ces mesures, on peut suivre, en fonction des jours post-opératoires, la diminution des efforts repris par la barre d'ostéosynthèse, qui est corrélée à l'augmentation de la rigidité du cal. L'appareil peut être pourvu de programmes permettant d'établir, en fonction des jours post-opératoires, des diagrammes d'évolution des efforts repris par la barre lors de sollicitations de référence, par exemple la flexion de la hanche en position couchée pour des fractures du tibia. Ces diagrammes sont comparés à des courbes préétablies afin de mettre en évidence des écarts par rapport à une évolution normale.

L'appareil peut éventuellement être pourvu d'une imprimante, afin de fournir un document résumant les mesures effectuées.

Le procédé, tel que revendiqué, sera plus aisément compris à l'aide des figures suivantes qui ne limitent en aucun cas l'invention.

La fig. 1 se rapporte à une barre de fixation ronde équipée de 5 ensembles de jauges de contrainte comprenant chacun une jauge.

La fig. 2 se rapporte à une barre de fixation ronde équipée de 5 ensembles de jauges de contrainte comprenant chacun deux jauges.

La fig. 3 se rapporte à une barre de fixation carrée équipé de 5 ensembles de jauges de contrainte comprenant chacun 4 jauges.

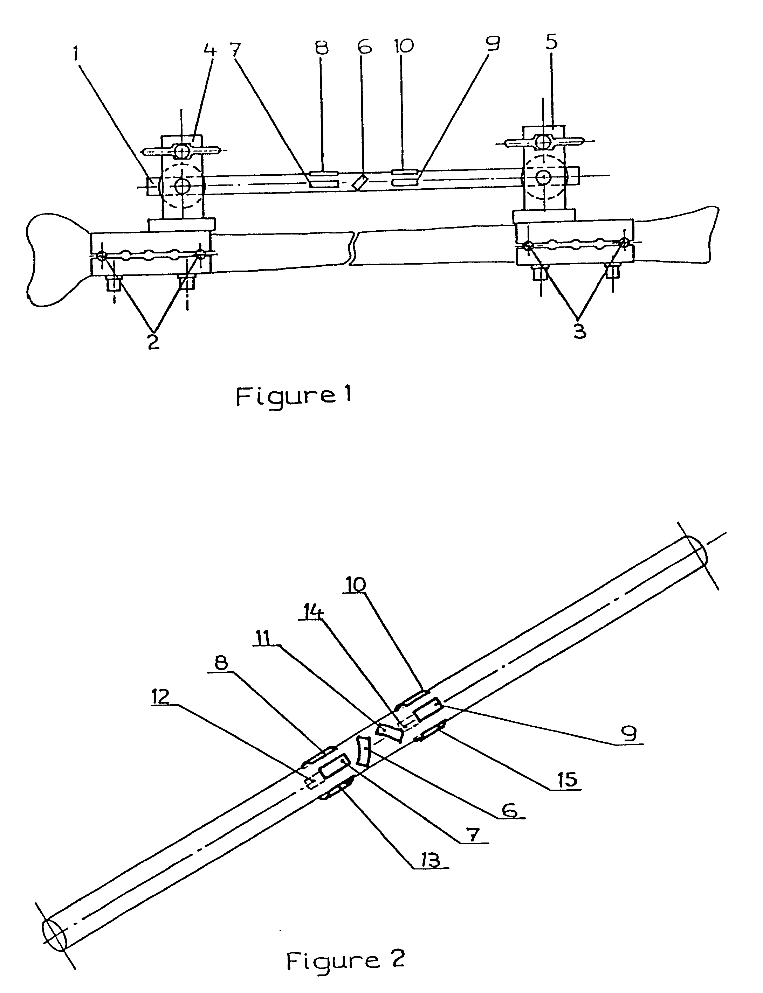

Suivant la figure 1, le montage orthopédique comprend une barre de fixation ronde 1, reliée, par l'intermédiaire des étaux 4 et 5, aux groupes de broches 2 et 3, constitués chacun de deux broches. La barre de fixation 1 est pourvue de 5 ensembles d'une jauge chacun. La jauge de contrainte 6 est disposée à 45° par rapport aux génératrices et mesure la torsion.

Les jauges 7 et 8 forment une paire d'ensemble d'une jauge chacun et sont disposées selon deux génératrices décalées de 90° dans une même section droite. Elles fournissent la valeur des composantes de la flexion en ce point.

Les jauges 9 et 10 forment une seconde paire d'ensembles d'une jauge chacun et sont disposées selon les mêmes génératrices que, respectivement les jauges 7 et 8, mais dans une section droite décalée d'une distance déterminée et connue. Elles fournissent la valeur des mêmes composantes de la flexion en ce second point.

Suivant la figure 2, seule la barre de fixation est représentée. Elle est reliée aux fragments osseux par l'intermédiaire de broches et d'étaux, de la même manière que représenté sur la figure 1.

Les jauges 6 et 11, constituant l'ensemble pour la mesure de la valeur de la torsion, sont disposées sur une même génératrice et forment entre elles un angle de 90°, et forment des angles de 45° avec les génératrices.

Les jauges, respectivement 7 et 12, et 8 et 13, sont disposées dans deux coupes longitudinales perpendiculaires de la barre et forment deux paires d'ensembles dans une même section transversale, mesurant la valeur des composantes de la flexion en ce point de la barre.

Les jauges respectivement 9 et 14, et 10 et 15 sont disposées de façon analogue, mais dans une section transversale décalée d'une distance bien déterminée et connue. Elles mesurent la valeur des mêmes composantes de la flexion en ce second point de la barre.

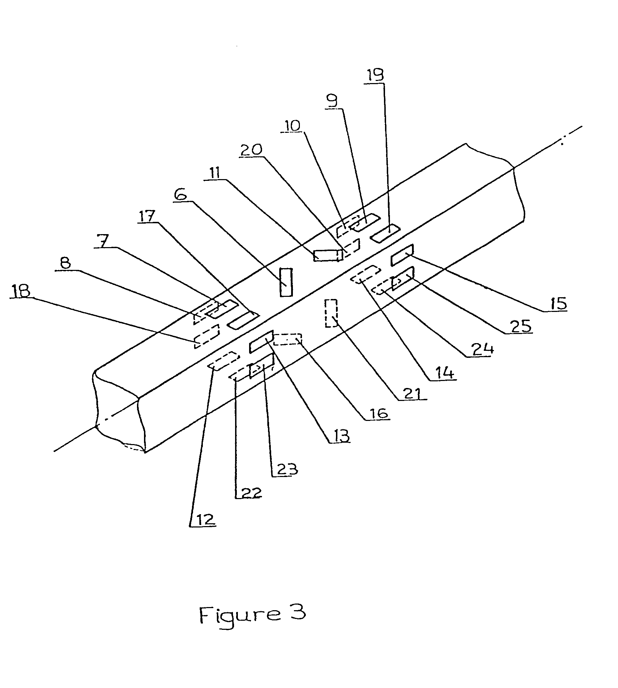

Suivant la figure 3, seule la partie de la barre comportant les jauges est représentée. La barre est fixée aux fragments osseux de façon analogue à la figure 1, et comporte des ensembles de 4 jauges. L'ensemble de mesure de la valeur de la torsion est constitué par les jauges 6, 11, 16 et 21.

La paire d'ensembles mesurant la valeur des composantes de la flexion en un premier point est constituée respectivement par les jauges 7, 12, 17 et 22, et par les jauges 8, 13, 18 et 23. Les jauges sont situées symétriquement par rapport à deux plans longitudinaux perpendiculaires, et dans une même section.

Les jauges respectivement 9, 14, 19 et 24, et 10, 15, 20 et 25 constituent une deuxième paire d'ensembles mesurant la valeur des mêmes composantes de la flexion en un deuxième point. Elles sont situées de façon analogue aux jauges de la première paire d'ensembles, mais dans une section transversale décalée d'une distance bien déterminée et connue.

Suivant une réalisation préférentielle du procédé revendiqué, la détermination de la valeur de la torsion et de la flexion, au niveau des étaux, au départ des signaux provenant des ensembles de jauges, et la comparaison de ces valeurs pouvant provoquer des glissements dans les étaux, se font en continu à l'aide d'un appareil de mesure et d'interprétation des résultats par un traitement logiciel.

Un exemple de réalisation consiste à utiliser un micro-ordinateur équipé des accessoires électroniques nécessaires à l'amplification et la conversion digitale des signaux provenant des jauges.

Ce micro-ordinateur est pourvu des programmes nécessaires pour effectuer un échantillonnage rapide des signaux des jauges, calculer la valeur de la torsion et de la flexion au niveau des étaux, et comparer, à chaque échantillonnage, ces valeurs aux valeurs pouvant provoquer des glissements dans les étaux, en tenant compte d'un facteur de sécurité déterminé par l'opérateur avant la séance de mesure.

**Revendications**

1. Procédé de contrôle de la stabilité d'un montage orthopédique effectué à l'aide d'une fixation externe et comprenant au moins une barre de fixation reliée à au moins deux broches au moyen d'étaux, caractérisé par l'utilisation d'une barre de fixation pourvue de 5 ensembles de jauges de contrainte d'au moins une jauge chacun; un ensemble mesurant la torsion de la barre et deux paires d'ensembles mesurant deux composantes perpendiculaires de la flexion de la barre en deux points de celle-ci, par la détermination, à l'aide de ces jauges de contrainte de la torsion et de la flexion au niveau des étaux qui relient la barre de fixation aux broches, et par la comparaison des niveaux de torsion et de flexion déterminés à ceux pouvant provoquer des glissements dans les étaux.

2. Procédé de contrôle de la stabilité d'un montage orthopédique effectué à l'aide d'une fixation externe, suivant la revendication 1, caractérisé en ce que les 5 ensembles de jauges de contrainte comprennent chacun de 1 à 4 jauges.

3. Procédé de contrôle suivant les revendications 1 et 2, caractérisé en ce que la détermination de la torsion et de la flexion au niveau des étaux est effectuée en continu à l'aide d'un appareil de mesure et d'interprétation des résultats par un traitement logiciel.

**Patentansprüche**

1. Verfahren zur Kontrolle der Stabilität einer orthopädischen Montage, bewirkt mit Hilfe einer äusserlichen Befestigung und enthaltend mindestens einen Befestigungsstab, der an mindestens zwei Zapfen mit Hilfe von Schraubstöcken befestigt ist, gekennzeichnet durch die Verwendung von einem Befestigungsstab, der versehen ist mit 5 Aufbauten von Beanspruchungsmessstäben von jeweils mindestens einem Messstab, wobei ein Aufbau die Torsion des Stabes misst und zwei Paare von Aufbauten zwei senkrechte Komponenten der Biegung des Stabes an zwei Punkten von diesem messen, durch die Bestimmung, mit Hilfe von diesen Beanspruchungsmessstäben, der Torsion und der Biegung auf der Höhe der Schraubstöcke, die den Befestigungsstab mit den Zapfen verbinden und durch den Vergleich der Höhen der Torsion und der Biegung bestimmt bei denjenigen, die Rutschen in den Schraubstöcken hervorrufen können.

2. Verfahren zur Kontrolle der Stabilität einer orthopädischen Montage, bewirkt mit Hilfe einer äusserlichen Befestigung nach Anspruch 1, dadurch gekennzeichnet, dass die fünf Aufbauten der Beanspruchungsmessstäbe jeweils von 1 bis 4 Messstäbe enthalten.

3. Verfahren zur Kontrolle nach Anspruch 1 oder 2, dadurch gekennzeichnet, dass die Bestimmung der Torsion und der Biegung auf der Höhe der Schraubstöcke ununterbrochen mit Hilfe eines Apparates zum Messen und zur Interpretation der Ergebnisse durch eine logische Behandlung bewirkt wird.

## Claims

1. A method of controlling the stability of an orthopaedic assembly carried out by means of an external fixture and comprising at least one clamping bar connected to at least two pins by means of clamps, characterised by the use of a clamping bar provided with 5 sets of strain gauges having at least one gauge each; one set measuring the torsion of the bar und two pairs of sets measuring two perpendicular components of the deflection of the bar at two places thereon, by determining by means of these strain gauges the torsion and deflection at the level of the clamps which connect the clamping bar to the pins and by comparing the levels of torsion and deflection thus determined with those able to induce sliding of the clamps.

2. A method of controlling the stability of an orthopaedic assembly carried out by means of an external fixture according to Claim 1, characterised in that the 5 sets of strain gauges each comprise from 1 to 4 gauges.

3. A control method according to Claims 1 and 2, characterised in that the determination of the torsion and deflection at the level of the clamps is carried out continuously by means of a device for measuring and interpreting the results by software processing.

5

Figure 1

Figure 2

Figure 3